(19) Europäisches Patentamt
European Patent Office
Office européen des brevets

(11) **EP 3 663 707 A1**

(12) **EUROPEAN PATENT APPLICATION**

(43) Date of publication:
**10.06.2020 Bulletin 2020/24**

(51) Int Cl.:
*G01B 11/16* (2006.01)          *A61B 5/06* (2006.01)
*G01D 5/353* (2006.01)          *G02B 6/02* (2006.01)
*A61B 34/20* (2016.01)

(21) Application number: **18210936.3**

(22) Date of filing: **07.12.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **VAN DUSSCHOTEN, Anna Hendrika
5656 AE Eindhoven (NL)**
• **HORIKX, Jeroen Jan Lambertus
5656 AE Eindhoven (NL)**
• **'T HOOFT, Gert Wim
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **METHODS AND SYSTEMS FOR SENSING SHAPE OF AN OPTICAL FIBER SENSOR**

(57)    The present invention relates to a method of sensing shape of an optical fiber sensor having an optical fiber, the optical fiber having embedded therein a plurality of fiber cores including a central fiber core extending along a longitudinal center axis of the optical fiber and at least one outer fiber core arranged in a radial distance from the longitudinal center axis and helically wound around the longitudinal center axis with a spin rate. The method comprises acquiring, from an optical distributed strain measurement along the plurality of fiber cores, a bend phase signal indicative of bend strain along the optical fiber sensor and a twist phase signal indicative of twist strain along the optical fiber sensor, and reconstructing the shape of the optical fiber sensor from the bend phase signal and the twist phase signal by relating the bend phase signal to curvature along the optical fiber sensor using a first scaling factor $f_{bend}$ and by relating the twist phase signal to a twist rate using a second scaling factor $f_{twist}$. At least one of the first scaling factor $f_{bend}$ and the second scaling factor $f_{twist}$ is taken as being dependent on position along the optical fiber sensor, so as to take into account of at least one of: a varying radial distance of at least one of the plurality of fiber cores from the longitudinal center axis along the optical fiber sensor and a varying spin rate of the at least one outer fiber core along the optical fiber sensor. Further, an optical shape sensing system is described.

FIG.1

**Description**

FIELD OF THE INVENTION

The present invention generally relates to the field of optical shape sensing using a spun multi-core optical fiber. In particular, the present invention relates to a method, a system and a computer program for sensing shape of an optical fiber sensor having an optical fiber, wherein the optical fiber has embedded therein a plurality of fiber cores including a central fiber core extending along a longitudinal center axis of the optical fiber and at least one outer fiber core arranged in a radial distance from the longitudinal center axis and helically wound around the longitudinal center axis with a spin rate.

BACKGROUND OF THE INVENTION

Optical shape sensing (OSS) is a useful technology to reconstruct a three-dimensional shape of a device, in particular elongated device. In case of medical appliances, such a device may be an interventional device, e.g. a catheter or guidewire, which is partially inserted into a patient's body and thus cannot be directly viewed with the user's eyes. With optical shape sensing using an optical shape sensing fiber integrated in the interventional device, the three-dimensional shape of the interventional device can be known and thus be made "visible" up to the tip of the device. The sensed shapes of the medical devices can be overlaid on X-ray images or a pre-operative CT scan. In this way a physician can navigate devices during a procedure without the need of X-ray tracking.
US 8,773,650 and US 9, 784,569 provide an overview about optical shape sensing. The whole contents of these documents is incorporated herein by reference.
In optical shape sensing, distributed strain and temperature signals are obtained from back-scattered spectra obtained with an interrogator system that incorporates interferometers. This is done for an optical fiber sensor with, for example, three outer fiber cores helically wound around a fourth core, which is in the center of the fiber.
The responses of the fiber cores to strain and temperature are measured as phase differences of the optical signals from the interferometers, as a function of delay position along the fiber sensor. The phase differences are obtained with respect to a reference measurement in which the fiber sensor is in a well-defined shape, for example a completely straight shape. From the phase differences of the fiber cores, the strain and temperature differences can be deduced for each fiber core. The strain signals will be the sum of bend strain in two orthogonal directions, as well as twist strain and axial strain, which is strain in the longitudinal direction of the optical fiber. From these four position dependent quantities, the shape of the fiber sensor can be reconstructed.
For high accuracy shape sensing, accurate knowledge of the properties of the optical fiber sensor is needed in the shape reconstruction model used in reconstructing the shape of the fiber sensor. Due to manufacturing tolerances, the fiber sensor properties cannot be simply taken from the specification of the fiber sensor so that the fiber sensor properties cannot be known with the required accuracy from the manufacturer. Therefore, accurate values for the fiber sensor properties need to be determined for each individual fiber sensor in a calibration process. In the calibration process, the fiber sensor is placed in several predetermined shapes with e.g. known curvature or with a strictly 2-dimensional layout, after which data is recorded with a shape sensing system. Dedicated algorithms are subsequently used to deduce the various fiber sensor properties. US 8,531,655 and WO 2016/099976 A1 describe calibration methods for various properties of optical fiber sensors.
A multi-core spun fiber sensor can be manufactured by rotating the preform during the fiber draw process. The rotational speed of the preform in combination with the drawing speed determines the spin rate of the outer fiber cores. The spin rate can for example be 50 turns per meter. Limitations in the manufacturing process will give variations in the spin rate, resulting in a non-constant spin rate even over the length of one and the same fiber sensor. For accurate shape sensing with multi-core spun fibers, the spin rate (also referred to as intrinsic twist rate) of the fiber sensor is an important property that should be calibrated.
The spin rate or intrinsic twist rate is the twist rate of the fiber sensor in the reference measurement, which is typically a straight shape of the optical fiber sensor, where no external twist is applied. The reference measurement is part of the calibration process. Any future measurement on the optical fiber sensor in arbitrary shapes will be compared to the reference measurement in order to obtain strain differences from which the shape can be deduced.
US 8,773,650 describes a method of how to calibrate the spin rate of a fiber sensor. Typically, the fiber sensor is placed in a shape with bend in only one plane, for example by creating a spiral on a flat surface. The bend strain differences obtained with this shape will show an oscillating behavior for the outer fiber cores. The radial distance of the outer fiber core to the longitudinal center axis of the optical fiber will determine the amplitude of the oscillations. The frequency of the oscillations is directly related to the twist rate applied along the fiber sensor. In order to obtain the intrinsic twist rate or spin rate, signals need to be compensated for the differences in torsion ("external twist") created by torque differences between the reference measurement and the flat spiral measurement.

The accuracy of the reconstructed shape is essential to the usefulness of the shape sensing technology. Firstly, the model description that converts interferometric signals into shape needs to be correct in order to obtain high accuracy. Further, as mentioned before, also the accuracy of the sensor properties determined in the calibration process directly limits the accuracy of the reconstructed shape. With longer sensor lengths and more demanding shape conditions (smaller bend radii, higher external twist) the requirements on the accuracy of the calibration outputs become more demanding.

The methods of shape sensing according to the prior art are still improvable in terms of accuracy.

SUMMARY OF THE INVENTION

It is an object to provide a method of a sensing shape of an optical fiber with increased accuracy in comparison with the prior art.

It is a further object of the present invention to provide a system of shape sensing with improved shape sensing accuracy.

In a first aspect of the present invention, it is provided a method of sensing shape of an optical fiber sensor having an optical fiber, the optical fiber having embedded therein a plurality of fiber cores including a central fiber core extending along a longitudinal center axis of the optical fiber and at least one outer fiber core arranged in a radial distance from the longitudinal center axis and helically wound around the longitudinal center axis with a spin rate, the method comprising

acquiring, from an optical distributed strain measurement along the plurality of fiber cores, a bend phase signal indicative of bend strain along the fiber sensor and a twist phase signal indicative of twist strain along the fiber sensor, reconstructing the shape of the fiber sensor from the bend phase signal and the twist phase signal by relating the bend phase signal to curvature along the fiber sensor using a bend scaling factor and by relating the twist phase signal to a twist rate using a twist scaling factor,

wherein at least one of the bend scaling factor and the twist scaling factor is taken as being dependent on position along the fiber sensor, so as to take into account of at least one of: a varying radial distance of at least one of the plurality of fiber cores from the longitudinal center axis along the fiber sensor and a varying spin rate of the at least one outer fiber core along the fiber sensor.

In shape sensing, phase differences are obtained from the back-reflection spectra with respect to a reference measurement. The change in phase difference (phase signal) gives the strain. In reconstructing the shape of the sensed fiber sensor, the bend phase signal at a position z along the fiber sensor is related to the curvature at this position z. In the relation between the bend phase signal and curvature, a parameter is used which is defined as the scaling factor between the bend phase signal and the curvature. Similarly, the change in twist phase difference (twist phase signal) and the twist rate is correlated by a parameter which is defined as the scaling factor between the twist phase signal and the twist rate of the fiber sensor.

In the prior art, the bend scaling factor and the twist scaling factor are assumed to be constants. In the shape sensing methods according to the prior art, it has been recognized that the fiber core distance may vary from fiber core to fiber core, and this variation has been calibrated. However, the present invention recognizes that the fiber core distance may not only vary from fiber core to fiber core and from fiber sensor to fiber sensor, but may also vary in longitudinal direction of one fiber sensor. According to the method of the present invention, at least one of the bend scaling factor and the twist scaling factor is taken as being dependent on position along the fiber sensor. Thereby, the method according to the present invention is enabled to take into account of a varying radial distance of at least one of the plurality of fiber cores from the longitudinal center axis along the fiber sensor and/or a varying spin rate of the at least one outer fiber core along the fiber sensor. Accordingly, the bend and/or twist scaling factors is/are not considered to be a constant any more, but to be a position dependent parameter dependent on the position along the fiber sensor. The present invention extends the reconstruction models described in the prior art with the advantage of an increased accuracy of the reconstructed shape of the fiber sensor.

A further advantage is that manufacturing tolerances on the variation of fiber core radial distance and the variation of spin rate within the fiber sensor may be relaxed so that manufacturing yield may be increased.

Also the spin rate may vary along one and the same fiber core due to limitations in the manufacturing process. However, in the prior art, this is not taken into account in the twist scaling factor.

In an embodiment, the method according to the present invention may also take into account the variation of the spin rate along the fiber sensor in the twist scaling factor. Accordingly, accuracy of shape reconstruction may be further increased and manufacturing tolerances on fiber spin rate variations may be relaxed.

According to an embodiment, both the bend scaling factor and the twist scaling factor may be as being dependent on position along the fiber sensor.

In this embodiment, the reconstruction model is further improved in terms of accuracy of shape reconstruction of the fiber sensor.

In a further embodiment, the bend scaling factor may be taken as function of the position dependent radial distance of at least one of the plurality of fiber cores from the longitudinal center axis along the fiber sensor. Thereby, variations of the radial distance of at least one fiber core along the fiber sensor is taken into account. It is to be noted here, that the at least one fiber core may also be the central fiber core extending along the longitudinal center axis of the optical fiber. In the ideal case the radial distance of the central fiber core from the longitudinal center axis is 0 along the fiber sensor, but may deviate from 0 due to manufacturing tolerances in the manufacturing process of the optical fiber.

The bend scaling factor may be taken as being proportional to $a(z)$, wherein $a(z)$ is the position dependent radial distance of at least one of the plurality of fiber cores from the longitudinal center axis of the fiber sensor.

The proportionality factor between the bend scaling factor and $a(z)$ may be obtained from a calibration of the fiber sensor and is assumed not to vary along the length of the fiber sensor so that the proportionality factor maybe calibrated for one fiber sensor of a plurality of fiber sensors of a same manufacturing batch.

Further, the twist scaling factor may be taken as function of the position dependent radial distance of the at least one outer fiber core from the longitudinal center axis along the fiber sensor. Thus, when calculating twist from the twist phase signal, variations of the radial distance of one or more of the fiber cores are taken into account in the reconstruction model with increased shape reconstruction accuracy.

Preferably, the twist scaling factor may be taken additionally as function of the position dependent spin rate of the at least one outer fiber core along the fiber sensor. In this embodiment, shape reconstruction accuracy is further improved when calculating twist (torsion) of the fiber sensor.

The twist scaling factor may be taken as being proportional to $a^2(z)\dfrac{d\vartheta_{\text{int}}}{dz}(z),$ wherein $a(z)$ is the the position dependent radial distance of the at least one outer core from the longitudinal center axis of the fiber sensor along the fiber sensor, and $\dfrac{d\vartheta_{\text{int}}}{dz}(z)$ is the position dependent spin rate of the at least one outer fiber core along the fiber sensor. The proportionality factor may be assumed to be constant along the fiber sensor.

The present invention does not only extend the reconstruction model for reconstructing the shape of the fiber sensor, but also provides a calibration of the position dependency of the bend scaling factor and/or twist scaling factor.

In an embodiment, the method may further comprise calibrating at least one of the position dependent radial distance of at least one of the plurality of fiber cores and the position dependent spin rate of the at least one outer fiber core to obtain a calibration of at least one of the bend and twist scaling factors. Thus, the invention enables knowledge of the fiber sensor properties as they may vary along the fiber sensor.

The calibrating may include using a normalization of at least one of the bend and twist scaling factors, wherein the normalization of the bend scaling factor is given as the quotient of the position dependent bend scaling factor and an average of the bend scaling factor along the fiber sensor, and the normalization of the twist scaling factor is given as the quotient of the position dependent twist scaling factor and an average of the twist scaling factor along the fiber sensor.

The averages of the first and second scaling factors may be obtained by a 'conventional' calibration known in the art. The normalized bend scaling factor and normalized twist scaling factor instead are position dependent and are obtained by calibrating the position dependent radial distance and/or the position dependent spin rate. Per definition, the average of the normalized bend scaling factor and the average of the normalized twist scaling factor along the fiber sensor is 1. Using normalized scaling factors simplifies calibration of the scaling factors.

Calibrating of the position dependent spin rate may comprise applying a shape with twist to the optical fiber sensor and using an iterative process starting with the twist scaling factor equalling an average of the twist scaling factor along the optical fiber sensor.

As will be described below, an iterative process for calibrating the internal spin rate of the at least outer fiber core using a shape of the fiber sensor with external twist (torsion) leads to accurate determination of the spin rate of the at least one outer fiber core.

Calibrating the position dependent radial distance may be performed in various ways.

In an embodiment, calibrating the position dependent radial distance may comprise applying a shape with known curvature along the optical fiber sensor. When using a normalization of the bend scaling factor, the calibration process may start with setting the normalized bend scaling factor to 1 in an iterative process.

Calibrating the position dependent radial distance may comprise applying axial strain in direction of the longitudinal center axis to the optical fiber sensor between two positions along the optical fiber sensor.

Another way of calibrating the position dependent radial distance may comprise, in a first shape measurement, applying a first spiral shape to the fiber sensor in one plane with a first amount of twist, and in a shape second measurement, applying a second spiral shape to the fiber sensor with a second amount of twist in the one plane, wherein the second amount of twist is substantially different from, e.g. higher than, the first amount of twist.

In the first shape measurement, the spin rate and the normalized twist scaling factor may be calculated without taking into account the position dependency of the radial distance of the at least one of the plurality of fiber cores. In the second shape measurement, the second spiral shape may be reconstructed using the spin rate and the normalized twist scaling factor obtained in the first shape measurement, and the variation of the fiber core radial distance may be deduced from the out-of-plane behaviour of the reconstructed shape and the variation of the twist difference of the reconstructed shape in the second shape measurement to the reconstructed shape in the first shape measurement.

As will be described herein, the three embodiments above for calibrating the position dependent radial distance give similar results.

In all of the afore-mentioned calibration processes, filtering can be applied to reduce the effect of noise.

A further embodiment of the method may comprise calibrating one of the bend and twist scaling factors, and calculating the other of the bend and twist scaling factor from the calibrated bend or twist scaling factor.

Calculating the bend scaling factor from the calibrated twist scaling factor or calculating the twist scaling factor from the calibrated bend scaling factor has the advantage that the number of shapes needed for the full calibration of a single fiber sensor can be reduced, and thus the time needed for the calibration of the fiber sensor will be lower. As will be described herein, the quotient of the twist scaling factor to the square of the bend scaling factor depends on the intrinsic spin rate of the at least one outer fiber core and some other parameters that can be considered constant in a fiber batch so that it would be principally sufficient to calibrate the internal spin rate only.

In another embodiment, the bend and twist scaling factors are calibrated separately. In this embodiment, the number of calibration shapes may not be reduced, but also in this embodiment, the above-mentioned relationship between bend scaling factor and twist scaling factor can be used as a control parameter to check or increase the accuracy on the bend scaling factor or twist scaling factor.

According to a second aspect of the present invention, a system for sensing shape of an optical fiber sensor having an optical fiber is provided, the optical fiber having embedded therein a plurality of fiber cores including a central fiber core extending along a longitudinal center axis of the optical fiber and at least one outer fiber core arranged in a radial distance from the longitudinal center axis and helically wound around the longitudinal center axis with a spin rate, the system comprising

an optical measuring unit configured to optically measure distributed strain along the plurality of fiber cores to acquire a bend phase signal indicative of bend strain along the fiber sensor and a twist phase signal indicative of twist strain along the fiber sensor,

a shape reconstruction unit configured to reconstruct the shape of the fiber sensor from the bend phase signal and the twist phase signal by relating the bend phase signal to curvature along the fiber sensor using a bend scaling factor and by relating the twist phase signal to a twist rate using a twist scaling factor,

the shape reconstruction unit being further configured to take at least one of the bend scaling factor and the twist scaling factor as being dependent on position along the fiber sensor, so as to take into account of at least one of: a varying radial distance of at least one of the plurality of fiber cores from the longitudinal center axis along the fiber sensor and a varying spin rate of the at least one outer fiber core along the fiber sensor.

The system according to the present invention has the same or similar advantages as the method according to the first aspect. It shall be understood that the claimed system has similar and/or identical preferred embodiments as the claimed method, in particular as defined in the dependent claims and as disclosed herein.

According to a third aspect of the present invention, a computer program is provided which comprises program code means for causing a computer to perform the steps of the method according to the first aspect, when the program is carried out by the computer, as well as a non-transitory computer-readable recording medium that stores therein a computer program product, which, when executed by a processor, causes the method according to the first aspect to be performed. The computer may be comprised by or may be a processor of the system according to the second aspect.

BRIEF DESCRIPTION OF THE DRAWINGS

These and other aspects of the invention will be apparent from and elucidated with reference to the embodiments described hereinafter. In the following drawings

Fig. 1 shows an embodiment of parts of a system for shape sensing of an optical fiber sensor;

Fig. 2 shows a piece of length of a spun optical fiber for use in the present invention;

Fig. 3 shows a graph of an example of a position dependent internal spin rate of an outer optical fiber core obtained in a calibration process;

Fig. 4 shows an example of a flat spiral shape of an optical fiber sensor in a calibration process;

Fig. 5 shows a diagram of fiber core radial distance variation along the length of the optical fiber obtained using three different calibration methods;

Fig. 6 shows a diagram of an example of a shape of an optical fiber sensor used in simulations performed to

**EP 3 663 707 A1**

investigate the effect of position dependency of bend and twist scaling parameters in shape reconstruction;

Fig. 7A and B show diagrams of simulation results for in-plane tip error (Fig. 7A) and out-of-plane tip error (Fig. 7B) in shape reconstruction as function of range in cladding diameter variation; and

Fig. 8A and B show diagrams of the dependency of the twist scaling parameter on internal spin rate (Fig. 8A) and on bend scaling parameter (Fig. 8B).

## DETAILED DESCRIPTION OF THE INVENTION

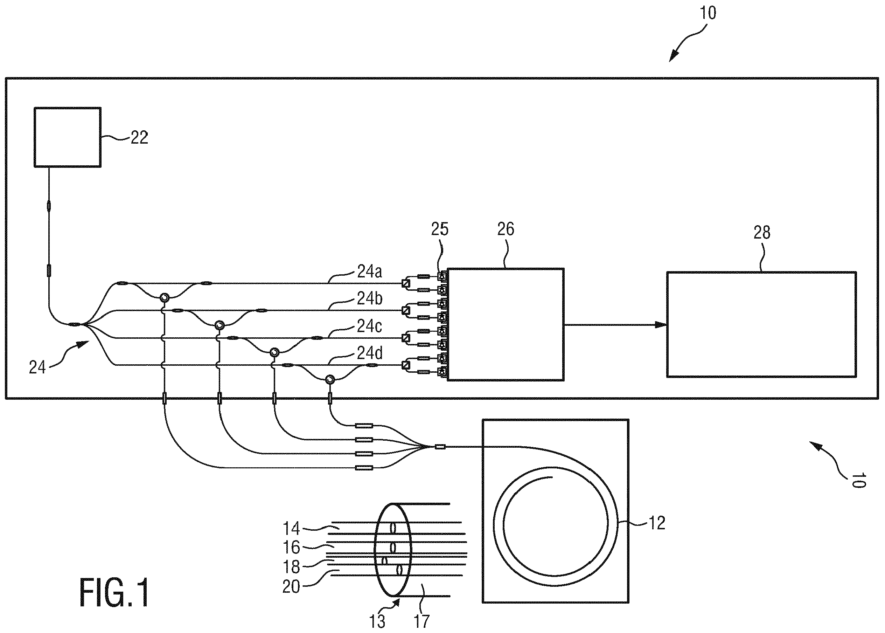

In the following, embodiments of a method and a system are described with which shape sensing of an optical fiber sensor can be carried out with high accuracy. Shape reconstruction using an optical fiber sensor may be performed by an optical shape sensing system, an embodiment of which will be first described with reference to Fig. 1.

Fig. 1 shows parts of an optical shape sensing system configured as a multi-channel OFDR-based distributed strain-sensing system for sensing a spun multi-core optical fiber sensor 12. The optical fiber sensor 12 comprises an optical fiber 13 having embedded in a cladding 17 a plurality of fiber cores 14, 16, 18, 20, in the example shown four fiber cores with one center fiber core 16 and three outer fiber cores 14, 18, 20.

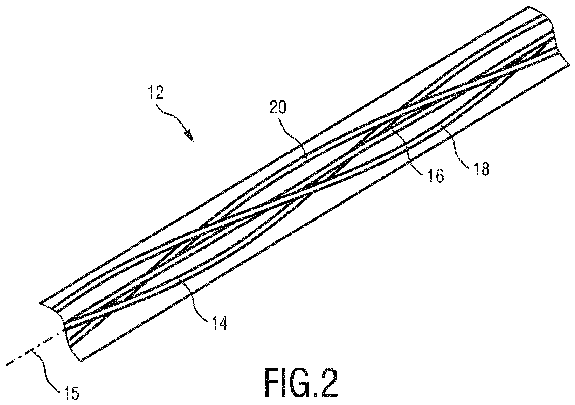

As shown in Fig. 2, the outer fiber cores 14, 18, 20 are helically wound around the center fiber core 16, the latter extending along a longitudinal center axis 15 of the fiber sensor 12.

With reference again to Fig. 1, the optical shape sensing system 10 comprises a tunable light source 22 which can be swept through a range of optical frequencies. The light emitted by the light source 22 is coupled into an interferometric interrogator 24 having four channels 24a, 24b, 24c, 24d, wherein each of the fiber cores 14, 16, 18, 20 is assigned one of the channels 24a, 24b, 24c, 24d. When the tunable light source 22 is swept through a range of optical frequencies, each channel 24a, 24b, 24c, 24d is simultaneously measured, and the resulting interference pattern from each channel 24a, 24b, 24c, 24d is routed to data acquisition electronics 26 via respective photodetectors 25. Each channel 24a, 24b, 24c, 24d is processed independently. The distributed strain measurements recorded using the multiple-channel OFDR system from the fiber cores 14, 16, 18, 20 may then be exported for use for further processing, as indicated by a processing or calculation unit 28, in particular for reconstructing the shape of the fiber sensor 12.

The light source 22 and the interferometric interrogator 24 including the data acquisition electronics 26 are part of or form an optical measuring unit configured to optically measure a distributed strain along the plurality of fiber cores 14, 16, 18, 20 to acquire a bend phase signal indicative of bend strain along the fiber sensor 12 and a twist phase signal indicative of twist strain along the fiber sensor 12, as described hereinafter. Unit 28 may form a reconstruction unit.

Light from the tunable light source 22 is coupled into each of the fiber cores 14, 16, 18, 20 of the optical fiber sensor 12, and the reflected or back-scattered light is made to interfere with light from the light source 22 that has travelled along a respective reference path in the channels 24a, 24b, 24c, 24d of the interrogator 24. When the frequency of the light source 22 is swept linearly in time, the interference between the light that is coming from a single fixed scattering point on a fiber core of the optical fiber sensor 12 and the reference light creates a detector signal in the respective detector 25 that has a constant frequency, this frequency being proportional to the difference of the travelled time of the light along the measurement path and the reference path. As the propagation velocity of the light and the length of the reference path are known, the position of the scattering point in the fiber sensor can be computed from the observed frequency. Back-scattering of the light in the fiber cores may be based on fiber Bragg gratings (not shown) or on Rayleigh-scattering.

Since multiple scatterers are present in the measurement fiber sensor 12 along each of the fiber cores 14, 16, 18, 20, the detector signal will be a superposition of different frequencies, each frequency indicative of the position of the respective scatterer along the respective fiber core. The Fourier transform of the detector signal (a "scattering profile") can be computed. In graphs of the amplitude and phase of the Fourier transform signal, the amplitude and phase of the different frequencies that are present in the detector signal (which correspond to different scatterer positions) will be shown at their respective positions along the horizontal axis of the graph.

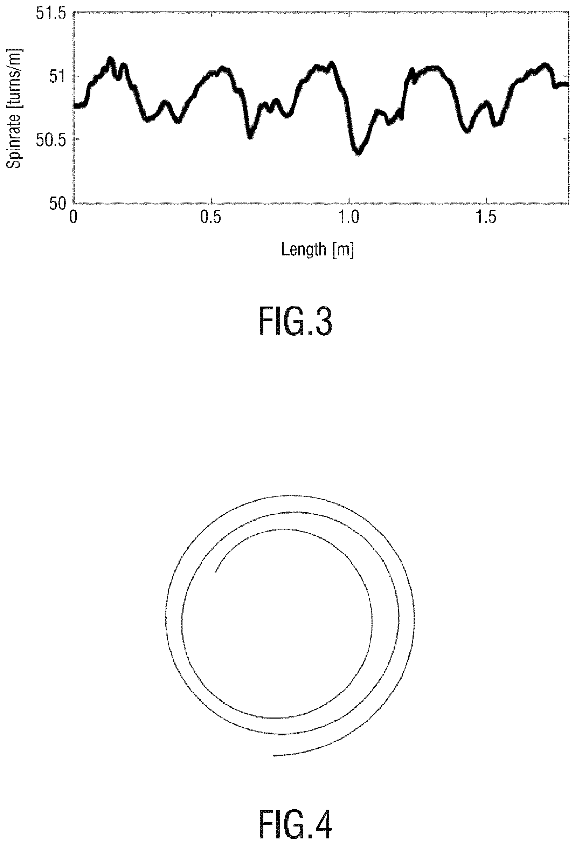

The amplitude and phase of the scattered light can be affected by external influences acting on the fiber sensor 12. For example, when the fiber sensor 12 is deformed by external stresses (e.g. as shown for a fiber sensor in Fig. 1 or 4, which is exemplarily shown as being shaped to a spiral), or when the temperature of the fiber sensor is modified, effects will be seen on the phase and/or amplitude of the scattering profile. From the comparison of the scattering profile of the fiber sensor actually measured with the scattering profile of the same fiber sensor in a reference state, information can be obtained about the external influences on the fiber sensor 12 as a function of position along the fiber sensor 12, i.e. the fiber sensor 12 can be used for distributed sensing.

The system 10 is capable of detecting optical path length changes of each fiber core 14, 16, 18, 20 of the spun multi-core fiber sensor 12 simultaneously over a range of positions along the fiber sensor 12, through a comparison of the scattering profiles of each fiber core 14, 16, 18, 20 of an actual "shape" measurement and a "reference" measurement. In the "reference" measurement, the fiber sensor 12 typically is in an unstrained, straight, shape.

Considering two arbitrary points located in the sensing region of the fiber sensor 12, a change of optical path length between these two points for a chosen fiber core of the fiber cores 14, 16, 18 or 20 when a shape measurement and an unstrained reference measurement are compared manifests itself as a change of the phase difference between these two points in the scattering profiles of the shape and the reference of this fiber core. If these points are chosen to be close to each other, the change of optical path length will be proportional to the strain of the fiber core in the vicinity of these points, while the change of phase difference is proportional to the derivative of the phase difference between the scattering profiles of shape and reference measurement. It follows that the local strain signal of a fiber core is proportional to the derivative of the difference in phase of the scattering profiles of the fiber core between the strained state and an unstrained reference state of that core.

The outer fiber cores 14, 18, 20 of the optical fiber sensor 12 are helically wound around the longitudinal center axis 15 with a spin rate which is typically determined by the manufacturing process of the optical fiber. For example, the multi-core spun optical fiber can be manufactured by rotating the preform during the fiber draw process. The rotational speed of the preform in combination with the drawing speed determines the final intrinsic twist rate or spin rate of the optical fiber. For example, the spin rate can be 50 turns per meter. Limitations in the process will give variations in the spin rate, resulting in a non-constant spin rate even over the length of the fiber cores. For accurate shape sensing with spun multi-core fibers, the spin rate (intrinsic twist rate) of the fiber sensor is an important property that should be calibrated. The spin rate obtained by calibration is also the twist rate of the fiber sensor in the reference measurement, which is typically a straight shape measurement, in which there is no external twist applied to the fiber sensor. In other words, in the reference measurement, external twist rate as measured should be the same as the intrinsic twist rate of the fiber sensor. The reference measurement is part of the calibration process. Any future measurement on the shape sensing fiber sensor will be compared to the reference measurement in order to obtain strain differences from which the shape of the fiber sensor as actually measured can be deduced.

A typical calibration process of the intrinsic spin rate may be as follows. The fiber sensor 12 is placed in a shape with bend in only one plane, for example by creating a spiral on a flat surface, for example as shown in Fig. 1. The bend strain differences obtained with this shape will show an oscillating behaviour for the outer fiber cores. The radial distance of the respective outer fiber core to the longitudinal center axis 15 will determine the amplitude of the oscillations. The frequency of the oscillations is directly related to the twist rate of the fiber due to the internal twist rate and the external twist applied to the fiber sensor. In order to obtain the spin rate (intrinsic twist rate), signals need to be compensated for the differences in torsion ('external twist') created by torque differences between the reference measurement and the flat spiral measurement.

The accuracy of the reconstructed shape is essential to the usefulness of the shape sensing technology. This requires firstly that the model description or reconstruction model that converts interferometric signals into shape needs to be correct in order to obtain high accuracy. Secondly, also the accuracy of the determination of the fiber sensor properties in the calibration process directly limits the accuracy of the shape reconstruction. With longer sensor lengths and more demanding shape conditions, e.g. smaller bend radii, higher external twist, the requirements on the accuracy of the calibration outputs become more demanding.

As described before, shape reconstruction of the optical fiber sensor 12 is performed by calculating the shape of the fiber sensor 12 from the position dependent strain signals measured for the fiber cores 14, 16, 18, 20 inside the optical fiber 13. For example, bending the optical fiber sensor 12 in a plane defined by a fiber core and the longitudinal center axis 15 will result in a strain on that fiber core if that fiber core is not exactly in the longitudinal center axis of the fiber:

$$\varepsilon_{\text{bend}} \overset{\text{def}}{=} \frac{\Delta l}{l} = \frac{a}{r} \qquad\qquad (1)$$

where $\varepsilon_{\text{bend}}$ is the strain experienced by the fiber core arranged at a distance $a$ from the longitudinal center axis 15, due to a bend with a radius $r$. The bend strain is measured here relative to the straight unstrained situation. Due to manufacturing limitations the fiber core distance $a$ may be different for the various outer fiber cores and needs to be calibrated. Also the central fiber core (fiber core 16) will not be exactly in the center axis 15 of the fiber sensor 12 and will experience some strain due to bend. Also this distance needs to be calibrated.

In case the fiber sensor has a different torsion (external twist) compared to the reference shape, strain due to the external twist will be measured. The length of an outer fiber core in a spun fiber is:

$$l = \sqrt{l_0{}^2 + l_0{}^2 a^2 \left(\frac{d\vartheta}{dz}\right)^2} \qquad\qquad (2)$$

where $l_0$ is the length of the central fiber core and $d\vartheta/dz$ is the spin rate of the fiber sensor 12 (in radians per length unit).

The strain $\varepsilon_{\text{twist}}$ on an outer fiber core with external twist applied is then:

$$\varepsilon_{\text{twist}} \stackrel{\text{def}}{=} \frac{l_{\text{no twist}} - l_{\text{twist}}}{l_{\text{no twist}}} \approx a^2 \frac{d\vartheta_{\text{int}}}{dz} \frac{d\vartheta_{\text{ext}}}{dz} \qquad (3)$$

The latter approximation is only valid for low external twist and small fiber core radial distances from the center axis.

In shape sensing, phase differences with respect to a reference measurement are obtained. The change in phase difference gives the strain. With respect to bend strain, the bend phase difference $\frac{d\Delta\varphi_{\text{bend}}(z)}{dz}$ is related to the curvature by:

$$-\frac{d\Delta\varphi_{\text{bend}}(z)}{dz} = \frac{4\pi n \xi a}{\lambda} \kappa \stackrel{\text{def}}{=} f_{\text{bend}} \kappa \qquad (4)$$

where $\lambda$ is the central wavelength of the laser scan range used for interrogating the fiber sensor, $n$ is the effective refractive index of the fiber core under consideration, $a$ is the distance of the fiber core to the longitudinal center axis, $\xi$ is a strain-optic number and $\kappa$ is the curvature. The parameter $f_{\text{bend}}$ is defined as the scaling factor (bend scaling factor) between the phase derivative and the curvature.

The relation between the twist phase difference $\frac{d\Delta\varphi_{\text{twist}}(z)}{dz}$ and the externally applied twist rate is as follows:

$$\frac{d\Delta\varphi_{\text{twist}}(z)}{dz} = -\frac{4\pi n \beta a^2 \frac{d\vartheta_{\text{int}}}{dz}}{\lambda} \frac{d\vartheta_{\text{ext}}}{dz} \stackrel{\text{def}}{=} f_{\text{twist}} \frac{d\vartheta_{\text{ext}}}{dz} \qquad (5)$$

where $\beta$ is a strain-optic number and $d\vartheta_{\text{int}}/dz$ is the spin rate of the fiber sensor. The parameter $f_{\text{twist}}$ is defined as the scaling factor between the phase derivative and the change in externally applied twist angle $\frac{d\vartheta_{\text{ext}}}{dz}$ (twist rate).

Up to here, a method of sensing shape of an optical fiber sensor 12 having an optical fiber 13, the optical fiber 13 having embedded therein a plurality of fiber cores 14, 16, 18, 20 including a central fiber core 16 extending along a longitudinal center axis 15 of the optical fiber 13 and at least one outer fiber core 14, 18 or 20 arranged in a radial distance from the longitudinal center axis and helically wound around the longitudinal center axis 15 with a spin rate has been described. The method comprises acquiring, from an optical distributed strain measurement along the plurality of fiber cores 14, 16, 18, 20, a bend phase signal, e.g. the bend phase difference $\frac{d\Delta\varphi_{\text{bend}}(z)}{dz}$, indicative of bend strain along the fiber sensor 12, and a twist phase signal, e.g. the twist phase difference $\frac{d\Delta\varphi_{\text{twist}}(z)}{dz}$, indicative of twist strain along the fiber sensor 12. Then, the shape of the fiber sensor is reconstructed from the bend phase signal and the twist phase signal by relating the bend phase signal to curvature along the fiber sensor 12 using a bend scaling factor, e.g. $f_{\text{bend}}$ as defined in equation (4), and by relating the twist phase signal to a twist rate using a twist scaling factor, e.g. $f_{\text{twist}}$ as defined in equation (5).

According to the principals of the present invention, the bend scaling factor $f_{\text{bend}}$ and the twist scaling factor $f_{\text{twist}}$ are no longer taken as position-independent constants, i.e. independent of the position along the fiber sensor 12, but as position dependent parameters, i.e. as parameters being dependent on the position along the fiber sensor 12. Since the bend scaling factor $f_{\text{bend}}$ and the twist scaling factor $f_{\text{twist}}$ are functions of the radial distance of each fiber core from the longitudinal center axis 15 and the twist scaling factor in addition on the spin rate, the position dependent bend and twist scaling factors take into account of a variation of the radial fiber core distance from the longitudinal center axis (also optionally of the central fiber core 16) even along the fiber sensor 12 and, preferentially, also a variation of the internal spin rate along the fiber sensor 12. This means, according to the principals of the present invention, not only a variation of the fiber core radial distance from fiber core to fiber core, but also in longitudinal direction along the fiber core is taken into account, and preferentially also the variation of the internal twist rate along the outer fiber core are taken into account.

This means that the parameter $f_{bend}$ becomes dependent on the position along the sensor: $f_{bend}(z)$. Similarly, the twist scaling factor $f_{twist}$ becomes a position dependent parameter: $f_{twist}(z)$.

An additional variation in $f_{twist}(z)$ is given when considering the internal spin rate as being position dependent as described above.

Position dependent bend and twist scaling factors applied in the reconstruction model may have the advantage of an increased accuracy of the reconstructed shape. A further advantage may be that manufacturing tolerances on fiber core radial distance from the center axis of the fiber and on spin rate variation may be relaxed and manufacturing yield may be increased.

The provision of position dependent scaling factors requires to calibrate these factors, as will be described next.

Basically, the bend scaling parameter $f_{bend}$ and the twist scaling parameter $f_{twist}$ may be put into a relationship so that it is possible to calculate $f_{bend}$ from $f_{twist}$ or to calculate $f_{twist}$ from $f_{bend}$. To this end, the following equation can be used:

$$\frac{f_{twist}}{f_{bend}^2} = -\frac{\lambda \beta}{4 \pi n \xi^2} \frac{d\vartheta_{int}}{dz} \qquad (6)$$

$\lambda$ is the scan center wavelength of the scan wavelength range and is known because it is a measurement setting, and $d\vartheta_{int}/dz$ is calibrated separately. The constants $\beta$, $\xi$ and $n$ will not differ much between fiber sensors within a single manufacturing batch. Once the product $\beta\xi^2 n$ is known for the batch, equation (6) can be used to calculate $f_{bend}$ from $f_{twist}$ or $f_{twist}$ from $f_{bend}$. Thus, the number of shapes needed for the full calibration of a single fiber sensor may be reduced, and thus the time needed for a calibration of a fiber sensor will be lowered. Otherwise, when the same number of calibration shapes is kept, then equation (6) can be used as a control parameter to check the calibration accuracy on $f_{bend}$ or $f_{twist}$, and/or equation (6) can be used to increase the accuracy of $f_{bend}$ and $f_{twist}$, by calculating and applying one calibration value for the product $\beta\xi^2 n$ for fiber sensors within one batch.

In the following, some types of calibration processes are described for calibrating a fiber sensor 12 taking into account a position dependent bend scaling factor $f_{bend}$ and/or position dependent twist scaling factor $f_{twist}$ used in the extended shape reconstruction model according to the principles of the present invention. The extension of the reconstruction model requires additional outputs from the calibration process. These outputs are a position dependent fiber core radial distance variation $a(z)$ and/or a position dependent internal spin rate variation $d\vartheta_{int}(z)/dz$. In the following several methods to calibrate these variations will be described.

The extension of the reconstruction model according to the principles of the invention and the corresponding calibration process may be dealt with by introducing the position dependency in normalized factors:

$$f_{bend}(z) \overset{def}{=} \overline{f_{bend}}\, f_{bend,norm}(z)$$

$$f_{twist}(z) \overset{def}{=} \overline{f_{twist}}\, f_{twist,norm}(z) \qquad (7)$$

By definition, $f_{bend}$ is the average of $f_{bend}(z)$ over the length of the fiber sensor, and the average of $f_{bend,norm}(z)$ over the entire sensor is 1. Also by definition, $f_{twist}$ is the average of $f_{twist}(z)$ over the length of the fiber sensor, and the average of $f_{twist,norm}(z)$ over the entire fiber sensor is 1. Then:

$$f_{bend,norm}(z) = \frac{a(z)}{\overline{a(z)}} \qquad (8)$$

and:

$$f_{twist,norm}(z) = \frac{a(z)^2 \dfrac{d\vartheta_{int}(z)}{dz}}{\overline{a(z)^2 \dfrac{d\vartheta_{int}(z)}{dz}}} \qquad (9)$$

Equation (8) may be derived from equation (4), and equation (9) may be derived from equation (5).

Conventional calibration methods as, for example, described in WO 2016/099976 A1, will give values for $\overline{f_{bend}}$

and $\overline{f_{twist}}$.

Next, calibration of the position dependent spin rate $d\vartheta_{int}/dz$ will be described. The position dependent spin rate $d\vartheta_{int}/dz$ of the reference shape is already calibrated separately using a different calibration shape or different calibration shapes. However, an improved spin rate measurement can only be obtained when the externally applied twist of this shape is known. The externally applied twist (external twist) of a shape is determined using the twist scaling factor $f_{twist}$ (z) that is to be calibrated here. Starting with $f_{twist}$ (z) = $\overline{f_{twist}}$ and following an iterative process, an accurate spin rate measurement can be obtained. Fig. 3 gives an example of the position dependent spin rate thus obtained in the calibration process. Fig. 3 shows the intrinsic spin rate in turns per meter as function along the length of the fiber sensor in meters. In this example, the spin rate thus calibrated reveals a variation of the internal spin rate along the fiber sensor around 51 turns per meter over a length of about 1.8 m of the fiber sensor.

Next, calibration of the position dependent fiber core distances from the center axis will be described.

Due to the nature of the fiber drawing process, the variation in the fiber core distance from the center axis is assumed to scale 1:1 with the variation of the diameter of the cladding 17 (Fig. 1) of the optical fiber 13. The cladding 17 of the optical fiber 13 has embedded therein the fiber cores 14, 16, 18, 20. For the same reason, the normalized variation of the fiber core radial distance from the center axis which determines the normalized bend twist factor $f_{bend,norm}$ (z) is assumed to be the same for all fiber cores within one fiber sensor.

Three embodiments of calibration methods for calibrating the position dependent fiber core radial distance will be described. The first method is based on a curvature measurement, the second method is based on an axial strain measurement, and the third method is based on a twist measurement. The three methods are independent and should yield substantially the same result (within small tolerances).

In the first method, a shape with constant curvature along its full length is applied to the fiber sensor and $f_{bend,norm}$ *(z)* is set to 1. The variation in curvature observed in the reconstructed shape scales with the variation in fiber core radial distance from the center axis.

In the second method, an axial force is applied to a section of the fiber sensor by fixating it between two points. The axial force F along this section of the fiber sensor will be independent of position, as the net force of an arbitrarily chosen part of the fiber sensor must remain zero in a static situation. The force of gravity may be neglected if the axial force F is large enough. The local strain $\varepsilon_{axial}$ experienced by the fiber sensor will be proportional to the local stress, which is the force F divided by the cross-sectional area:

$$\varepsilon_{axial} = \frac{F}{\pi\, R_0^2\, E_g} \qquad\qquad (10)$$

$R_0$ is the radius of the cladding and the proportionality constant $E_g$ is the Young's modulus of the optical fiber glass. The strain is inversely proportional to the square of the radius of the cladding of the optical fiber. The relative variation of the cladding diameter can be obtained from analyzing the variation of axial strain on the fiber sensor. The relative variation of the cladding diameter is assumed to be equal to the relative variation of the radial distance of an outer fiber core from the center axis. It is to be noted that in equation (10) the effect of the coating (not shown in Fig. 1 and 2) of the optical fiber 13 is neglected, which is acceptable, because the Young's modulus of the coating is much lower than the Young's modulus of the optical fiber glass.

In the third method, the calibration of the fiber core radial distance variation is performed using a twist applied to the optical fiber sensor. To this end, the fiber sensor is put into at least two different flat spiral shapes, one of which is shown in Fig. 4. Data for two shape measurements on the flat spiral shapes are recorded, wherein the first shape measurement is a measurement in a first shape of the fiber sensor with a low amount of externally applied twist, and the second shape measurement is a measurement in a second shape of the fiber sensor with a high amount of externally applied twist. The spin rate may be calculated from the flat spiral shape with low twist using

$$f_{twist,norm}(z) = \frac{d\vartheta_{int}(z)}{dz} \Big/ \overline{\frac{d\vartheta_{int}(z)}{dz}},$$ differently from equation (9). The shape of the flat spiral with high twist is reconstructed using this spin rate and the same $f_{twist,norm}$ (z) as was used for the flat spiral with low twist. Now it is assumed that the out-of-plane behavior of this shape with high twist is due to the fact that the fiber core radial distance variation was not taken into account in $f_{twist,norm}$ (z) as given in equation (9). The relative fiber core radial distance variation along the length of the fiber sensor 12 can be deduced from the variation in the out-of-plane behavior and the variation in the external twist difference between the two measurements. The relative fiber core radial distance variation along the fiber sensor as obtained in this way, in combination with the obtained spin rate variation, gives the final $f_{twist,norm}$ (z). The higher the twist difference between the two shape measurements, the more accurate the result will be.

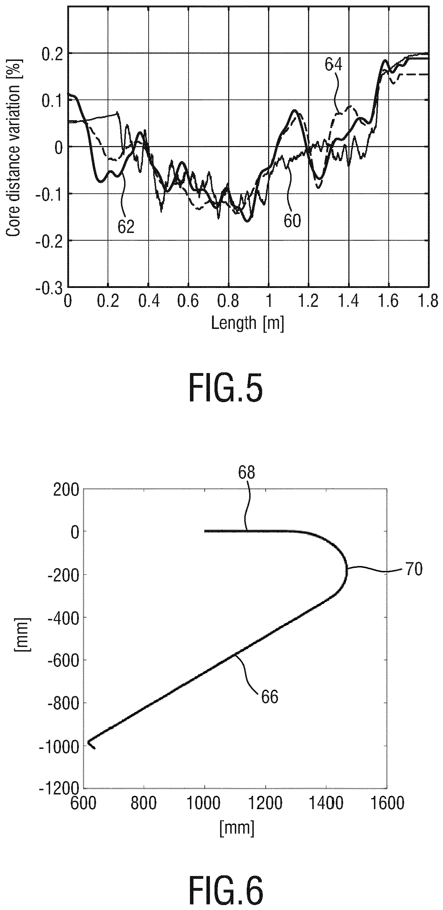

In all three methods described above filtering can be applied to reduce the effect of noise. Fig. 5 shows calibration results for the fiber core radial distance variation along the length of the fiber sensor using the three different calibration methods. Curve 60 shows the calibration results from the third method ('flat spirals'), curve 62 shows the calibration results from the second method ('axial strain'), and curve 64 shows the calibration results from the first method ('curvature'). As may be taken from Fig. 5, the three methods give substantially the same results. For the fiber sensor calibrated by the three methods, the range in variation in fiber core radial distance along the length of the fiber sensor is about 0.3%. For a fiber sensor with an average radial fiber core distance $\bar{a}$ = 35 $\mu$m, the 0.3% range corresponds to 0.1 $\mu$m variation in fiber core radial distance from the center axis along the fiber sensor.

In the following, simulations will be described which have been performed to investigate the effect of the position dependency of both the bend and twist scaling factors. The magnitude of the shape error due to inaccurate bend and twist calibration parameters depends very much on the shape of the fiber sensor which is to be reconstructed. Fig. 6 shows the shape that was used in the simulations. The shape has two straight portions 66, 68 and a bend 70 between the straight portions 66, 68. Shape reconstruction of the shape in Fig. 6 was performed for $f_{bend,norm}$ = 1 and $f_{twist,norm}$ = 1 (conventional reconstruction model) as well as for $f_{bend,norm}$ = $f_{bend,norm}$ (z) and $f_{twist,norm}$ = $f_{twist,norm}$ (z) according to the principles of the present invention. The difference in the reconstructed shapes gives the shape error. 97 fiber sensors have been investigated. Cladding diameter variation data along the fiber sensors (which scale 1:1 with the fiber core radial distance variation as described above) of the 97 fiber sensors was used. The effect of varying spin rate in the twist scaling factor was not simulated separately, but will give similar results.

An error in the bend scaling factor results in an in-plane-shape error. An error in the twist scaling factor results in an out-of-plane shape error. The latter error scales with the magnitude of the applied external twist. In the simulations, an external twist rate of 3 radiance/1.8 m is assumed, linearly distributed over the length of the respective fiber sensor.

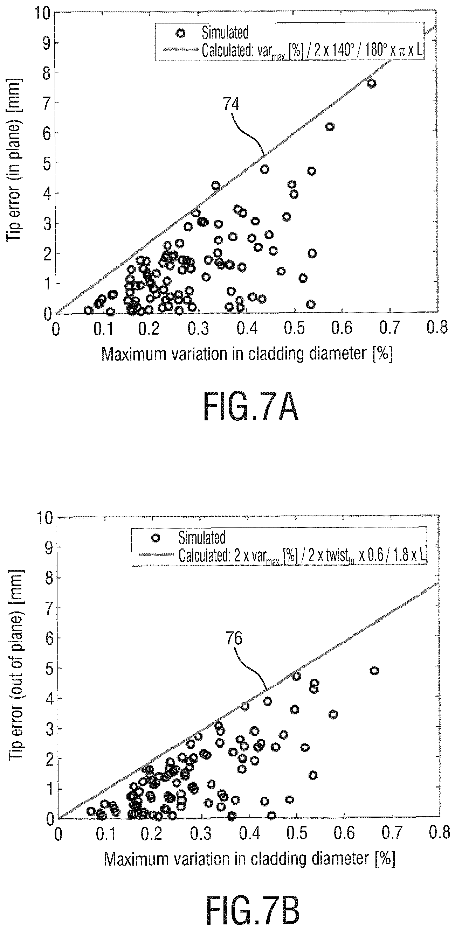

Fig. 7A and 7B show the results of the simulations for the in-plane shape error (Fig. 7A) and the out-of-plane shape error (Fig. 7B) at the tips of the fiber sensors, as a function of the range in cladding diameter variation along the fiber sensors. Each circle in Fig. 7A and B represents one fiber sensor. The maximum tip errors for a certain range of variation in cladding diameter are approximated by a straight line 74 and 76, respectively, which can be calculated from the shape properties shown in Fig. 6. It is to be noted here that the deviation of average cladding diameter at the position of the bend differs from fiber sensor to fiber sensor. Therefore, a variation in tip error occurs even for sensors with the same range of cladding diameter variation. It can be observed from the two graphs in Fig. 7A and 7B that for the shape in Fig. 6 and depending on the fiber sensor easily millimeters of shape accuracy can be gained by using position dependent fiber core radial distance. Using the position dependent spin rate in the twist scaling factor will increase the out-of-plane shape accuracy even more.

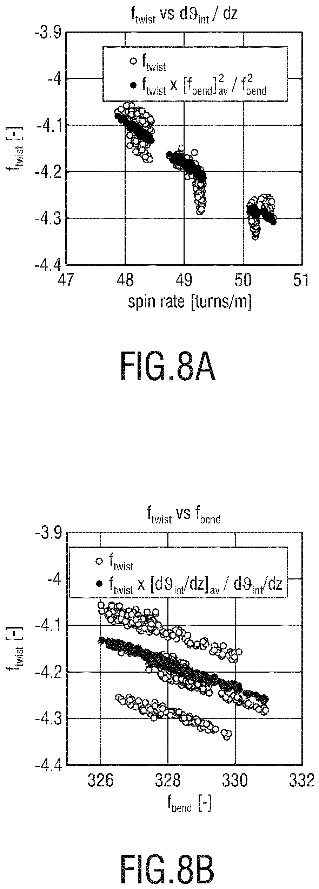

Next, the dependency of the twist scaling parameter on the fiber core radial distance and on the spin rate will be described. Sensor calibration data was obtained for more than 500 shape sensing fibers. The position dependency of $f_{bend}$, $f_{twist}$ and spin rate was removed by taking averages over the fiber sensor length. Figs. 8A and 8B give the results for the more than 500 sensors. In Fig. 8A, $f_{twist}$ is given as function of the spin rate. The bright grey dots show the expected linear dependency of $f_{twist}$ on the spin rate, which becomes more pronounced when the variation due to fiber core radial distance is taken out using $f_{bend}$ (black dots). Similarly, when looking at $f_{twist}$ as function of $f_{bend}$ in Fig. 8B, there is a quadratic dependency on $f_{bend}$ (see equation (6) above): The range in $f_{bend}$ is 1.5% while $f_{twist}$ varies over 3%. This dependency becomes more clear when the variation due to the spin rate is removed (black dots). As explained before, these relations can be used to calculate $f_{bend}$ from $f_{twist}$ without the need for calibration shapes for $f_{bend}$, or to calculate $f_{twist}$ from $f_{bend}$ without the need for calibration shapes for $f_{twist}$. These relations can also be used for control of the accuracy of the calibration output or to increase the accuracy of $f_{bend}$ and $f_{twist}$, by calculating and applying a batch calibration value for the product $\beta l \xi^2 n$.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Method of sensing shape of an optical fiber sensor (12) having an optical fiber (13), the optical fiber (13) having embedded therein a plurality of fiber cores (14, 16, 18, 20) including a central fiber core (16) extending along a longitudinal center axis (15) of the optical fiber (13) and at least one outer fiber core (14, 18, 20) arranged in a radial distance from the longitudinal center axis (15) and helically wound around the longitudinal center axis (15) with a spin rate, the method comprising

   acquiring, from an optical distributed strain measurement along the plurality of fiber cores (14, 16, 18, 20), a bend phase signal indicative of bend strain along the fiber sensor (12) and a twist phase signal indicative of twist strain along the fiber sensor (12),

   reconstructing the shape of the fiber sensor (12) from the bend phase signal and the twist phase signal by relating the bend phase signal to curvature along the fiber sensor (12) using a bend scaling factor and by relating the twist phase signal to a twist rate using a twist scaling factor,

   wherein at least one of the bend scaling factor and the twist scaling factor is taken as being dependent on position along the optical fiber sensor (12), so as to take into account of at least one of: a varying radial distance of at least one of the plurality of fiber cores (14, 16, 18, 20) from the longitudinal center axis along the optical fiber sensor (12) and a varying spin rate of the at least one outer fiber core (14, 18, 20) along the optical fiber sensor (12).

2. Method of claim 1, wherein the bend scaling factor is proportional to $a(z)$, wherein $a(z)$ is the position dependent radial distance of at least one of the plurality of fiber cores (14, 16, 18, 20) from the longitudinal center axis (15) of the optical fiber sensor (12).

3. Method of claim 1, wherein the twist scaling factor is taken as function of at least one of the position dependent radial distance of the at least one outer fiber core (14, 18, 20) from the longitudinal center axis (15) along the optical fiber sensor (12) and the position dependent spin rate of the at least one outer fiber core (14, 18, 20) along the optical fiber sensor (12).

4. Method of claim 3, wherein the twist scaling factor is proportional to $a^2(z)\dfrac{d\vartheta_{\text{int}}}{dz}(z),$ wherein $a(z)$ is the the position dependent radial distance of the at least one outer fiber core (14, 18, 20) from the longitudinal center axis (15) of the optical fiber sensor (12) along the optical fiber sensor (12) and $\dfrac{d\vartheta_{\text{int}}}{dz}(z)$ is the position dependent spin rate of the at least one outer fiber core (14, 18, 20) along the optical fiber sensor (12).

5. Method of claim 1, further comprising calibrating at least one of the position dependent radial distance of at least one of the plurality of fiber cores (14, 16, 18, 20) and the position dependent spin rate of the at least one outer fiber core (14, 18, 20) to obtain a calibration of at least one of the position dependent bend and twist scaling factors.

6. Method of claim 5, wherein the calibrating includes using a normalization of at least one of the position dependent bend and twist scaling factors, wherein the normalization of the bend scaling factor is given as the quotient of the position dependent bend scaling factor and an average of the bend scaling factor along the optical fiber sensor (12), and the normalization of the twist scaling factor is given as the quotient of the position dependent twist scaling factor and an average of the twist scaling factor along the optical fiber sensor (12).

7. Method of claim 5, wherein the calibrating of the position dependent spin rate comprises applying a shape with twist to the optical fiber sensor (12) and using an iterative process starting with the twist scaling factor equalling an average of the twist scaling factor along the optical fiber sensor (12).

8. Method of claim 5, wherein the calibrating of the position dependent radial distance comprises applying a shape with constant curvature along the optical fiber sensor (12).

9. Method of claim 5, wherein the calibrating of the position dependent radial distance comprises applying axial strain in direction of the longitudinal center axis (15) to the optical fiber sensor (12) between two positions along the optical fiber sensor (12).

10. Method of claim 5, wherein the calibrating of the position dependent radial distance comprises, in a first shape measurement, applying a first spiral shape to the optical fiber sensor (12) in one plane with a first amount of twist, and in a second shape measurement, applying a second spiral shape to the optical fiber sensor (12) with a second amount of twist in the one plane, wherein the second amount of twist is substantially different from the first amount of twist.

11. Method of claim 5, further comprising calibrating one of the bend and twist scaling factors, and calculating the other of the bend and twist scaling factor from the calibrated bend or twist scaling factor.

12. System for sensing shape of an optical fiber sensor (12) having an optical fiber (13), the optical fiber (13) having embedded therein a plurality of fiber cores (14, 16, 18, 20) including a central fiber core (16) extending along a longitudinal center axis (15) of the optical fiber (13) and at least one outer fiber core (14, 18, 20) arranged in a radial distance from the longitudinal center axis (15) and helically wound around the longitudinal center axis (15) with a spin rate, the system comprising
an optical measuring unit (24, 25, 26) configured to optically measure distributed strain along the plurality of fiber cores (14, 16, 18, 20) to acquire a bend phase signal indicative of bend strain along the optical fiber sensor (12) and a twist phase signal indicative of twist strain along the optical fiber sensor (12),
a shape reconstruction unit (28) configured to reconstruct the shape of the optical fiber sensor (12) from the bend phase signal and the twist phase signal by relating the bend phase signal to curvature along the optical fiber sensor (12) using a bend scaling factor and by relating the twist phase signal to a twist rate using a twist scaling factor,
the shape reconstruction unit (28) being further configured to take at least one of the bend scaling factor and the twist scaling factor as being dependent on position along the optical fiber sensor (12), so as to take into account of at least one of: a varying radial distance of at least one of the plurality of fiber cores (14, 16, 18, 20) from the longitudinal center axis (15) along the optical fiber sensor and a varying spin rate of the at least one outer fiber core (14, 18, 20) along the optical fiber sensor (12).

13. Computer program comprising program code means for causing a computer to carry out the steps of the method as claimed in claim 1 when said computer program is carried out on a computer.

FIG.1

FIG.2

FIG.3

FIG.4

FIG.5

FIG.6

FIG.7A

FIG.7B

FIG.8A

FIG.8B

Europäisches
Patentamt

European
Patent Office

Office européen
des brevets

# EUROPEAN SEARCH REPORT

Application Number

EP 18 21 0936

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/213874 A1 (INTUITIVE SURGICAL OPERATIONS [US]) 14 December 2017 (2017-12-14) | 1-13 | INV. G01B11/16 A61B5/06 |
| Y | * paragraph [0054] - paragraph [0091]; figures 1-17 * * paragraph [0092] - paragraph [0130]; figures 18-22,28,29,32,33,37 * ----- | 1-13 | G01D5/353 G02B6/02 ADD. A61B34/20 |
| X,D | US 9 784 569 B2 (INTUITIVE SURGICAL OPERATIONS [US]) 10 October 2017 (2017-10-10) | 1,12,13 | |
| Y | * column 12, line 18 - column 15, line 47; figures 12-23,27 * ----- | 1-13 | |
| Y | WO 2017/144667 A1 (KONINKLIJKE PHILIPS NV [NL]) 31 August 2017 (2017-08-31) * the whole document * ----- | 1-13 | |

TECHNICAL FIELDS
SEARCHED (IPC)

G01B
A61B
G01D
G02B

The present search report has been drawn up for all claims

| Place of search | Date of completion of the search | Examiner |
|---|---|---|
| Munich | 25 January 2019 | Burkart, Johannes |

EPO FORM 1503 03.82 (P04C01)

**ANNEX TO THE EUROPEAN SEARCH REPORT
ON EUROPEAN PATENT APPLICATION NO.** EP 18 21 0936

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2019

| Patent document cited in search report | Publication date | Patent family member(s) | Publication date |
|---|---|---|---|
| WO 2017213874 A1 | 14-12-2017 | NONE | |
| US 9784569 B2 | 10-10-2017 | BR 112012008347 A2 | 22-03-2016 |
| | | CN 102695938 A | 26-09-2012 |
| | | CN 104535009 A | 22-04-2015 |
| | | CN 104880159 A | 02-09-2015 |
| | | EP 2478331 A2 | 25-07-2012 |
| | | JP 5506935 B2 | 28-05-2014 |
| | | JP 2013505441 A | 14-02-2013 |
| | | RU 2012115444 A | 27-10-2013 |
| | | US 2011109898 A1 | 12-05-2011 |
| | | US 2014320846 A1 | 30-10-2014 |
| | | US 2017370705 A1 | 28-12-2017 |
| | | WO 2011034584 A2 | 24-03-2011 |
| WO 2017144667 A1 | 31-08-2017 | EP 3420306 A1 | 02-01-2019 |
| | | WO 2017144667 A1 | 31-08-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8773650 B
- US 9784569 B
- US 8531655 B
- WO 2016099976 A1